# EUROPEAN PATENT SPECIFICATION

(11) **EP 3 027 174 B1**
(45) Date of publication and mention of the grant of the patent: **24.07.2019**
(21) Application number: 13826764.6
(22) Date of filing: 10.12.2013
(51) Int. Cl.: A61K 9/16, A61K 9/20, A61K 9/48, A61K 31/137

(54) **PHARMACEUTICAL COMPOSITIONS OF FINGOLIMOD**
PHARMAZEUTISCHE ZUSAMMENSETZUNGEN VON FINGOLIMOD
COMPOSITIONS PHARMACEUTIQUES À BASE DE FINGOLIMOD

(30) Priority: 29.07.2013 IN 3368CH2013
(43) Date of publication of application: 08.06.2016
(73) Proprietor: Aizant Drug Research Solutions Private Limited, Andhra Pradesh, 500014 Hyderabad (IN)
(72) Inventor: ALLURI, Pavan Kumar, Hyderabad 500 014 Andhra Pradesh (IN); MYLAMALA, Subhash Chandra Bose, Hyderabad 500 014 Andhra Pradesh (IN); DONTIKA, Nagaraju, Hyderabad 500 014 Andhra Pradesh (IN); THUMMISETTY, Mastanaiah, Hyderabad 500 014 Andhra Pradesh (IN); KANDARAPU, Raghupathi, Hyderabad 500 014 Andhra Pradesh (IN); RUDRARAJU, Varma S, Hyderabad 500 014 Andhra Pradesh (IN)
(74) Representative: Hamm&Wittkopp Patentanwälte PartmbB
(86) International application number: PCT/IB2013/060770
(87) International publication number: WO 2015/015254

(56) References cited:
- WO-A1-2012/135561
- WO-A2-2010/055028
- GB-A- 2 400 318

## Description

### Field of Invention

The invention relates to a stable pharmaceutical composition comprising fingolimod, a pharmaceutically acceptable salt thereof or a phosphate derivative as an active ingredient and its preparation.

### Background of Invention

Fingolimod (2-amino-2-[2-(4-octylphenyl)ethyl]-propane-1,3-diol) is a sphingosine-1 phosphate (S1P) receptor modulator. Fingolimod is metabolized by sphingosine kinase to the active metabolite, fingolimod phosphate. Chemically, it is know as (2-amino-2-[2-(4-octylphenyl)ethyl]-propane-1,3-diol) and is structurally represented as given below.

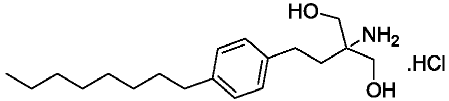

Fingolimod is approved for treating patients with relapsing forms of multiple sclerosis by reducing the frequency of clinical exacerbations and to delay the accumulation of physical disability. Fingolimod sequesters lymphocytes in lymph nodes, preventing them from moving to the central nervous system for autoimmune responses in multiple sclerosis.

Fingolimod is currently marketed as an immediate release capsule for the treatment of multiple sclerosis under the trade name Gilenya® in the US. This formulation contains 0.5 mg equivalent of fingolimod base in the form of the hydrochloride salt and magnesium stearate, mannitol as inactive ingredients.

US 5,604,229 first disclose fingolimod and its pharmaceutically acceptable salts and process for its preparation. US 6,004,565 disclose method of manipulating lymphocyte traffic in a mammal by administering fingolimod hydrochloride.

US 8,324,283 disclose a solid pharmaceutical composition for oral administration comprising fingolimod and a sugar alcohol. The sugar alcohol may be mannitol, maltitol, inositol, xylitol or lactitol.

US 2008/0096972 disclose a pharmaceutical organic concentrate formulation comprising fingolimod or a salt thereof in an organic solvent of ethanol in propylene glycol.

US 2010/0040678 & US 2012/288559 discloses a pharmaceutical composition comprising fingolimod with a coating comprising polymers resins and metal oxides.

US 2010/0267675 discloses dosage forms containing fingolimod and one or more excipients selected from fillers, binders, disintegrants, lubricants, flow regulators, matrix formers, plasticizers, flavouring agents and sweeteners.

US 2011/0229501 disclose a pharmaceutical composition of hydrochloride salt of fingolimod in the form of a hydrate.

US2013/0034603 discloses a process of preparing a pharmaceutical composition of fingolimod comprising preparing an intimate admixture of fingolimod and a solid surfactant and optionally combining the admixture with one or more excipients.

US 2013/0095177 discloses a method of preparation of an intermediate containing fingolimod and excipients, having particles size of all the intermediate particles less than 250 µm and greater than 0.6µm.

US 2013/0102682 disclose an intermediate containing fingolimod and matrix material, wherein the fingolimod is present in the matrix material in the form of a solid solution.

US 2013/0102683 disclose a method of preparing an intermediate comprising melt processing fingolimod and a matrix former.

US 2012/0328664 disclose a concentrate for dilution comprising a SIP receptor modulator or agonist and propylene glycol.

WO 2013/091704 discloses a pharmaceutical composition comprising fingolimod, calcium lactate pentahydrate and optionally a lubricant.

WO2012/135561 discloses a solid oral pharmaceutical composition comprising fingolimod, a filler and a cyclodextrin as a stabilizer.

Preparation of pharmaceutical formulation of fingolimod is not an easy task as the drug itself either in its free form; in a pharmaceutically acceptable salt form or as a phosphate derivative possess properties that can cause processing problems during preparation. The stability and uniformity of pharmaceutical compositions containing fingolimod is heavily dependent on the choice of excipients used in the formulation, and the process by which the formulation is prepared. Fingolimod is unstable in presence of many excipients due to the reactivity of aminopropane-1,3-diol group and produce degradation products in the final formulation.

Even though prior art teaches about different fingolimod compositions, there still exists a need for stable pharmaceutical composition of the fingolimod. In particular, there is a need for a stable pharmaceutical composition of fingolimod, which are stable through out the shelf life. The present invention relates to a stable pharmaceutical composition of fingolimod and its preparation.

### Objective of the Invention

The object of the present invention is to provide a stable composition of Fingolimod or a pharmaceutically acceptable salt thereof.

Another object of the invention is to provide a stable pharmaceutical composition comprising fingolimod, wherein the total impurity of the composition is less than the total impurity of the composition containing sugar alcohol.

### Summary of Invention

Accordingly, the present invention provides a stable pharmaceutical composition comprising fingolimod, a pharmaceutically acceptable salt thereof or a phosphate derivative and a zwitterion as a stabilizer.

In yet another embodiment, the present invention provides a process for the preparation of stable pharmaceutical composition comprising fingolimod, a pharmaceutically acceptable salt thereof or a phosphate derivative and a zwitterion as a stabilizer.

### Detailed Description of the Invention

The embodiment of the present invention is to provide a stable pharmaceutical composition of fingolimod, which comprises fingolimod, a pharmaceutically acceptable salt thereof or a phosphate derivative and a zwitterion.

The zwitterion is used as a stabilizer in the composition and is present in an amount ranging from about 0.1 to 99.5 % by weight of total composition, preferably from 1 to 98.5 %, more preferably 5 to 98.5 % by weight of total composition. The weight ratio of Fingolimod to zwitterion is from 90:10 to 1:99.

The zwitterion according to the present invention is selected from an amino acid, a phospholipid, and a sulfobetaine (NS).

The non-limiting examples of amino acids as Zwitterion are selected from glycine, arginine, histidine, alanine, isoleucine, leucine, asparagine, lysine, aspartic acid, methionine, cysteine, phenylalanine, glutamic acid, threonine, glutamine, tryptophan, valine, ornithine, proline, selenocysteine, serine, tyrosine or a combination thereof. The preferable amino acids are glycine, leucine or a mixture thereof.

Zwitterion phospholipids constitute any phospholipid with ionizable groups where the net charge is zero. The non-limiting examples of zwitterion phospholipid are phosphatidyl choline, phosphatidyl ethanolamine, sphingomyeline, lysophatidylethanolamine, cerebrosides, dimyristoylphosphatidyl choline, dipalmitotylphosphatidyl choline, distearyloylphosphatidyl choline, dielaidoylphosphatidyl choline, dioleoylphosphatidyl choline, dilauryloylphosphatidyl choline, 1-myristoyl-2-palmitoyl phosphatidyl choline, 1-palmitoyl-2-myristoyl phosphatidyl choline, 1-palmitoyl-phosphatidyl choline, 1-stearoyl-2-palmitoyl phosphatidyl choline, dimyristoyl phosphatidyl ethanolamine, dipalmitoyl phosphatidyl ethanolamine, brain sphingomyelin, dipalmitoyl sphingomyelin, distearoyl sphingomyelin, and mixtures thereof. Preferably, the zwitterion phospholipid is phosphatidyl choline.

The non-limiting examples of amino acids of Sulfobetaine are Dimethylsulfonioacetate.

In another embodiment, the stable composition of Fingolimod further comprises one or more pharmaceutically acceptable excipients selected from diluent, binder, disintegrant, surafactant, glidant, lubricant and the like.

Fingolimod or its pharmaceutically acceptable salts or phosphate derivatives thereof, of the present invention may be in form of amorphous, crystalline or solvated form such as anhydrous, hydrate and the like.

The term "pharmaceutically acceptable salt" include inorganic or organic acids such as hydrochloric, hydrobromic, nitric, sulfuric, phosphoric, acetic, benzenesulfonic, benzoic, camphorsulfonic, citric, ethanesulfonic, fumaric, gluconic, glutamic, isethionic, lactic, maleic, malic, mandelic, methanesulfonic, mucic, oxalic, pamoic, pantothenic, succinic, tartaric, p-toluenesulfonic acid and the like.

The active ingredient, active agent and drug herein can be interchangeably used.

As used herein, "%" refers to the weight percent of a substance as it relates to the overall composition unless otherwise indicated.

The term "comprising", which is synonymous with "including", "containing", or "characterized by" here is defined as being inclusive or open-ended, and does not exclude additional, unrecited elements or method steps, unless the context clearly requires otherwise.

In a preferred embodiment, the present invention provides a pharmaceutical composition comprising fingolimod, a pharmaceutically acceptable salt thereof or a phosphate derivative and glycine or leucine as a stabilizer. The glycine used in the present invention is having a mean particle size less than 250µm, preferably less than 160µm. The d₉₀ of glycine particle size is less than 400µm, preferably less than 350µm. The leucine used in the present invention is having a mean particle size less than 350µm, preferably less than 250µm. The d₉₀ of leucine particle size is less than 600µm, preferably less than 500 µm.

In yet another embodiment, the present invention provides a pharmaceutical composition comprising fingolimod, a pharmaceutically acceptable salt thereof or a phosphate derivative and a zwitterion as a stabilizer, wherein the total impurity of the composition is less than the total impurity of the composition containing only sugar alcohol.

In another preferred embodiment, the present invention provides a pharmaceutical composition comprising fingolimod, a pharmaceutically acceptable salt thereof or a phosphate derivative and glycine or leucine or a mixture thereof as a stabilizer, wherein the total impurity of the composition is less than the total impurity of the composition containing only a sugar alcohol.

Suitable diluents according to the present invention are selected from microcrystalline cellulose, powdered cellulose, lactose (anhydrous or monohydrate), compressible sugar, fructose, dextranes, other sugars such as mannitol, sorbitol, lactitol, saccharose or a mixture thereof, siliconised microcrystalline cellulose, calcium hydrogen phosphate, calcium carbonate, calcium lactate or mixtures thereof. A preferred further diluent that also causes reduced sticking properties of tablets to the equipment used for tabletting is silica, preferably colloidal or fumed silica. Preferably, the diluent is selected from microcrystalline cellulose, lactose monohydrate or mixture thereof. The diluent is present in amount from about 10% to about 80%, preferably from about 5%, to about 50%, by weight of the composition.

Suitable binders according to the present invention are selected from polyvinyl pyrollidone, polyvinylpyrrolidone/vinyl acetate copolymer, polyvinyl alcohol, polymers of acrylic acid and its salts, starch, celluloses and celluloses derivatives like methylcellulose, ethyl cellulose, hydroxyethyl cellulose, hydroxyl propyl cellulose, ethylhydroxyethylcellulose, hydroxypropyl methylcellulose, carboxymethyl cellulose etc., maltrin, sucrose solution, dextrose solution, acacia, tragacanth, locust bean gum, gelatine, guar gum, starch, pregelatinised starch, partially hydrolysed starch, alginates, xanthan or polymethacrylate, or mixtures thereof. It is preferable to use a binder with good water solubility. Preferably, the binder is selected from hydroxypropyl cellulose and povidone. The binding agent is present in the composition in an amount of from about 1% to about 25%, preferably from about 1%, to about 15%, more preferably from about 1% to about 10% by weight of the composition.

Suitable lubricants according to the present invention are selected from stearic acid, magnesium stearate, calcium stearate, sodium lauryl sulphate, hydrogenated vegetable oil, hydrogenated castor oil, sodium stearyl fumarate, macrogols, or mixtures thereof. Preferably, the lubricant is selected from stearic acid, magnesium stearate, calcium stearate and sodium lauryl sulphate, more preferably from stearic acid, magnesium stearate and calcium stearate. The lubricant is present in an amount from about 0.5% to about 5% by weight of the composition.

Suitable disintegrants according to the present invention are selected from crospovidone, modified starches especially sodium starch glycolate, carmellose especially croscarmellose sodium, carboxymethylcellulose calcium and the mixture thereof. The disintegrant is present in the composition in an amount of from about 1% to about 20 %, preferably from about 1% to about 15%, more preferably from about 1% to about 10% by weight of the composition.

The composition can also comprises glidants such as colloidal silica (e. g. Aerosil®), magnesium trisilicate, powdered cellulose, starch, talc, and tribasic calcium phosphate.

Suitable surfactants according to the present invention are selected from cyclodextrin and its derivatives, lipophilic substances or any combination thereof. Non-limiting examples of surfactants include non-ionic, anionic, cationic, amphoteric or zwitterionic or any combination thereof. Non-ionic surfactant is preferable.

In another embodiment, the stable composition of the present invention is free of sugar alcohol.

In a preferred embodiment, the present invention provides a stable pharmaceutical composition comprising fingolimod or a pharmaceutically acceptable salt thereof or a phosphate derivative, 5 to 98.5% by weight of a zwitterion and one or more pharmaceutically acceptable excipients.

In another preferred embodiment, the present invention provides a stable pharmaceutical composition comprising fingolimod or a pharmaceutically acceptable salt thereof or a phosphate derivative, 5 to 98.5% by weight of a zwitterion selected from glycine, arginine, histidine, alanine, isoleucine, leucine, asparagine, lysine, aspartic acid, methionine, cysteine, phenylalanine, glutamic acid, threonine, glutamine, tryptophan, valine, ornithine, proline, selenocysteine, serine, tyrosine or a combination thereof and one or more pharmaceutically acceptable excipients.

In yet another preferred embodiment, the present invention provides a stable pharmaceutical composition comprising fingolimod or a pharmaceutically acceptable salt thereof or a phosphate derivative, 5 to 98.5% by weight of a zwitterion and at least one pharmaceutically acceptable excipient selected from
a) diluent selected from microcrystalline cellulose, lactose monohydrate or mixture thereof;
b) binder selected from hydroxypropyl cellulose or povidone;
c) lubricants selected from stearic acid, magnesium stearate or calcium stearate.
d) disintegrant selected from crospovidone, sodium starch glycolate, croscarmellose sodium,
e) glidant selected from colloidal silica or talc.

In one embodiment, the compositions of the present invention may be in the form of capsules, powders, granules, tablets, pills, lozenges, sachets, suppositories etc. The dosage form is preferably suitable for oral application. The compositions are preferably formulated in a unit dosage form, each dosage containing about 0.05 to about 20 mg, more usually about 0.1 to about 10 mg of fingolimod, most preferably about 0.2 to about 5mg of fingolimod. The term "unit dosage form" refers to physically discrete units suitable as unitary dosages for human subjects and other mammals, each unit containing a predetermined quantity of fingolimod calculated to produce the desired therapeutic effect, in association with a suitable pharmaceutical excipient. The pharmaceutical composition of the present invention is preferably a capsule, powder or granules in sachets.

Optionally, powder, cores/tablets can be coated with conventional materials used for film coating, i.e. as described in "Pharmaceutical Coating Technology", 1995, edited by Graham Cole. Film coating formulations usually contain the following components: polymer(s), plasticizer (s), colourant(s) /opacifier(s), vehicle(s). In film coating suspension the minor quantities of flavours, surfactants and waxes can be used. The polymers used in film coating are either cellulose derivatives, such as the cellulose ethers, or acrylic polymers and copolymers. Occasionally encountered are high molecular weight polyethylene glycols, polyvinyl pyrrolidone, polyvinyl alcohol and waxy materials.

Typical cellulose ethers are hydroxyethylcellulose, hydroxypropylcellulose, hydroxypropylmethylcellulose and methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration / dissolution of the film.

The commonly used plasticizers can be categorized into three groups: polyols (glycerol, propylene glycol and macrogols), organic esters (phthalate esters, dibutyl sebacetate, citrate esters, and triacetin), oils/glycerides (castor oil, acetylated monoglycerides, and fractionated coconut oil).

Colourants/opacifiers are classified into several groups: organic dyes and their lakes, inorganic colours, natural colours. Combination of different materials form each group can be combined in defined ratios. Film coating suspensions can be used as ready-to-make preparations which are available on the market.

Film coating dispersion can be prepared using solvents selected from water, alcohols, ketones, esters, chlorinated hydrocarbons and the like or mixture thereof.

A composition of coating suspension (calculated on dry material) is particularly preferred which comprises: 1-99% by weight of polymer, preferably 1- 95% of polymer; 1-50% by weight of plasticizer, preferably 1-40% of plasticizer; 0.1-20% of colourant/opacifier, preferably 0.1- 10% of colourant/opacifier, all the percentage are based on the total weight of coating material.
The present pharmaceutical compositions are prepared by known technological procedures, e.g. direct blending and capsule filing, direct compression, dry granulation or wet granulation. In the preparation of the compositions of fingolimod, the active ingredient will usually be mixed with an excipient or mixture of excipients, or diluted by an excipient or mixture of excipients, or enclosed within an excipient or mixture of excipients which may be in the form of a capsule, sachet, paper or other container. When the excipient serves as a diluent, it may be a solid, semisolid or liquid material which acts as a vehicle or medium for the fingolimod.

The composition of the invention can be produced by compressing a mixture of the drug substance of the invention with excipients. For example, one method for the production includes mixing fingolimod with the materials for the preparation by a suitable mixer, and followed by capsule filing or directly compressing the mixture to tablets. Other methods include a dry granulating step to produce granules using dry granulating machines or roller compacters, and a wet granulating step using water, ethanol and solutions containing binders, to produce granules for filling into capsules or compressing into tablets.

In one aspect, the present invention relates to a process for producing a pharmaceutical composition, comprising:
(a) mixing fingolimod or a pharmaceutical acceptable salts or a phosphate derivative thereof with zwitterion;
(b) optionally, granulating the mixture obtained in the step (a); and
(c) mixing the mixture from the step (a) or, optionally, from the step (b) with a lubricant,
(d) finally filing the mixture of step (c) to capsules or compressing into tablets.

In another aspect, the present invention relates to a process for producing a pharmaceutical composition, comprising:
(a) mixing fingolimod or a pharmaceutical acceptable salts or a phosphate derivative thereof with zwitterion;
(b) optionally, granulating the mixture obtained in the step (a); and
(c) optionally, mixing the mixture from the step (a) or (b) with one or more additional excipients like diluents, disintegrants, surfactants;
(d) lubricating the mixture of step (c); and
(e) finally filing the mixture of step (d) to capsules or compressing into tablets.

In another embodiment, at least 75 % of fingolimod is dissolved from the pharmaceutical composition in a 0.1N HCl + 0.2% sodium lauryl sulphate in 30 minutes, when dissolution is performed using USP apparatus I (basket), at a temperature of the dissolution medium of 37±0.5°C, speed of rotation of the basket 100 rpm and volume of the dissolution medium 500 ml. Preferably the drug release rate of the composition of the invention is more than 70% in 15 minutes, above 80%, e. g. 90%, over 30 minutes, and above 95% over 45 minutes.

The composition of the invention containing fingolimod is preferably administered once daily in an amount of 0.1 to 5 mg/day.

The pharmaceutical compositions of the present invention are useful in (a) treatment and prevention of organ or tissue transplant rejection, for example for the treatment of the recipients of heart, lung, combined heart-lung, liver, kidney, pancreatic, skin or corneal transplants, and the prevention of graft-versus-host disease, such as sometimes occurs following bone marrow transplantation; particularly in the treatment of acute or chronic alio- and xenograft rejection or in the transplantation of insulin producing cells, e.g. pancreatic islet cells; (b) treatment and prevention of autoimmune disease or of inflammatory conditions, e.g. chronic long term diseases, e.g. multiple sclerosis, arthritis (for example rheumatoid arthritis), inflammatory bowel disease, hepatitis, etc.; treatment and/or prevention of viral myocarditis and viral diseases caused by viral myocarditis, including hepatitis and AIDS. Multiple sclerosis takes several forms, with new symptoms occurring either in discrete attacks (relapsing forms) or slowly accumulating over time (progressive forms). As herein defined, multiple sclerosis refers, but is not limited to, relapsing remitting multiple sclerosis (RRMS) or primary progressive multiple sclerosis (PPMS), e.g. RRMS.

In one embodiment, a pharmaceutical composition comprising fingolimod or a pharmaceutically acceptable salt thereof of the invention has a comparable bioavailability to the commercial form of fingolimod. In one preferred embodiment, a pharmaceutical composition comprising fingolimod is bioequivalent to commercial dosage form of fingolimod.

The following experimental details are set forth to aid in understanding of the invention,

### Example 1

| **Ingredients** | **%w/w** |
|---|---|
| Fingolimod HCl | 1.16 |
| Glycine | 11.63 |
| Polyvinylpyrrolidone | 3.11 |
| Purified water | q.s. |
| Glycine | 83.06 |

| **Extragranular material** | |
|---|---|
| Talc | 1.04 |

**Procedure:** Fingolimod HCl, a part of glycine were mixed in water followed by addition of polyvinylpyrrolidone and remaining part of glycine. The wet mass was kept for drying at 50°C in oven for about 9hrs. The obtained dried granules were sized, mixed with talc and finally filled into capsule or compressed into tablet.

### Example 2

| **Ingredients** | **Example 2a** | **Example 2b** | **Example 2c** |
|---|---|---|---|
| | %w/w | %w/w | %w/w |
| Fingolimod HCl | 1.14 | 1.14 | 1.14 |
| Mannitol SD 200 | 61.15 | 83.16 | 75.83 |
| Glycine | 36.69 | 14.68 | 22.01 |
| Magnesium stearate | 1.02 | 1.02 | 1.02 |

**Procedure:** Mannitol SD-200 and glycine were mixed together, followed by co-shifting with Fingolimod HCl and mixing. The drug mixture was lubricated with magnesium Stearate and filled into capsule or compressed into tablet.

### Example 3

| **Ingredients** | **%w/w** |
|---|---|
| Fingolimod HCl | 1.14 |
| Glycine | 97.84 |
| Talc | 1.02 |

**Procedure:** Fingolimod HCl and glycine were co-shifted and mixed well. The drug mixture was mixed well with talc and then filled into capsule or compressed into tablet.

### Comparative Example:

| **Ingredients** | **%w/w** |
|---|---|
| Fingolimod HCl | 1.14 |
| Mannitol SD 200 | 97.84 |
| Magnesium stearate | 1.02 |

**Procedure:** Fingolimod HCl and Mannitol SD-200 co-shifted and mixed well followed by lubricating with magnesium stearate and then filled into capsule or compressed into tablet as described in US 8,324,283.

**Stability Study:** Stability study was conducted for active ingredient, compositions of the present invention and comparative composition. The study was conducted at 40°C/75% RH for one week.

| | **Example 1** | | |
|---|---|---|---|
| **Impurities** | Initial | After one week | |
| | Sample (% w/w) | Blend (% w/w) | Capsules (% w/w) |
| Impurity 1 | 0.03 | 0.04 | 0.04 |
| Impurity 2 | ND | ND | ND |
| Impurity 3 | ND | ND | ND |
| Impurity 4 | ND | ND | ND |
| Total Impurities | 0.13 | 0.12 | 0.14 |

| | **Example 2a** | | |
|---|---|---|---|
| **Impurities** | Initial | After one week | |
| | Sample (% w/w) | Blend (% w/w) | Capsules (% w/w) |
| Impurity 1 | 0.09 | 0.07 | 0.07 |
| Impurity 2 | 0.23 | 0.44 | 0.23 |
| Impurity 3 | ND | 0.19 | 0.33 |
| Impurity 4 | ND | 0.12 | 0.11 |
| Total Impurities | 0.47 | 0.85 | 0.78 |

| **Impurities** | **Example 3** | | |
|---|---|---|---|
| | Initial | After one week | |
| | Sample (% w/w) | Blend (% w/w) | Capsules (% w/w) |
| Impurity 1 | 0.05 | 0.05 | 0.04 |
| Impurity 2 | ND | ND | 0.05 |
| Impurity 3 | ND | ND | ND |
| Impurity 4 | ND | ND | ND |
| Total Impurities | 0.10 | 0.08 | 0.18 |

| | **API** | | **Comparative Example** | | |
|---|---|---|---|---|---|
| **Impurities** | Initial | After one week | Initial | After one week | |
| | Sample (% w/w) | Sample (% w/w) | Sample (% w/w) | Blend (% w/w) | Capsules (% w/w) |
| Impurity 1 | 0.05 | 0.05 | 0.05 | 0.05 | 0.08 |
| Impurity 2 | ND | ND | 0.40 | 0.84 | 0.47 |
| Impurity 3 | ND | ND | ND | 0.08 | 0.19 |
| Impurity 4 | ND | ND | ND | 0.05 | 0.09 |
| Total Impurities | 0.05 | 0.05 | 0.45 | 1.02 | 0.84 |

| | | | | | |
|---|---|---|---|---|---|
| ND: Not Detected | | | | | |

### Example 4

| **Ingredients** | **%w/w** |
|---|---|
| Fingolimod HCl | 1.16 |
| Leucine | 14.74 |
| Polyvinylpyrrolidone | 4.15 |
| Purified water | q.s. |
| Leucine | 78.9 |

| **Extragranular material** | |
|---|---|
| Talc | 1.04 |

**Procedure:** Fingolimod HCl, a part of Leucine were mixed in water followed by polyvinylpyrrolidone and remaining part of Leucine. The wet mass was kept for drying at 50°C in oven for about 9hrs. The obtained dried granules were sized, mixed with talc and finally filled into capsule or compressed into tablet.

### Example 5

| **Ingredients** | **%w/w** |
|---|---|
| Fingolimod HCl | 1.14 |
| Mannitol SD 200 | 53 |
| Leucine | 44.84 |
| Magnesium stearate | 1.02 |

**Procedure:** Mannitol SD-200 and leucine were mixed together, followed by co-shifting with Fingolimod HCl and mixing. The drug mixture was lubricated with magnesium Stearate and filled into capsule or compressed into tablet.

### Example 6

| **Ingredients** | **%w/w** |
|---|---|
| Fingolimod HCl | 1.14 |
| Leucine | 98.25 |
| Talc | 0.61 |

**Procedure:** Fingolimod HCl and leucine were co-shifted and mixed well. The drug mixture was mixed well with talc and then filled into capsule or compressed into tablet.

### Example 7

| **Ingredients** | **Mg/Tablet** | **%W/W** |
|---|---|---|
| **Intragranular material** | | |
| Fingolimod HCl | 0.56 | 1.17 |
| Glycine | 45.44 | 94.67 |
| Polyvinyl pyrrolidone(PVP) k29/32 | 1.5 | 3.13 |

| **Extra granular material** | | |
|---|---|---|
| Talc | 0.5 | 1.04 |
| **Total** | **48.00** | **100** |

**Procedure:.** Part of of Glycine and PVP was loaded in RMG, and mixed well followed by granulation with Fingolimod HCl and small amount of Glycine dissolved in water. The wet mass was dried to obtain granule, mixed well with extragranular MCC and talc and then filled into capsule or compressed into tablet.

### Example 8

| **Ingredients** | **Mg/Tablet** | **%W/W** |
|---|---|---|
| **Intragranular material** | | |
| Fingolimod HCl | 0.56 | 0.86 |
| Glycine | 22.72 | 34.95 |
| Micro crystalline cellulose (MCC) 101 | 22.72 | 34.95 |
| Polyvinyl pyrrolidone (PVP) k29/32 | 1.5 | 2.31 |
| Plasdone X1 10 | 7.5 | 11.54 |
| Purified water | QS | Q.s |

| **Extra granular material** | | |
|---|---|---|
| Micro crystalline cellulose (MCC) 101 | 9 | 13.85 |
| Talc | 1 | 1.54 |
| **Total** | **65** | **100** |

**Procedure:** Fingolimod HCl and small amount of Glycine was dissolved in water. Remaining amount of Glycine, PVP K, MCC, Plasdone XL 10 was loaded in RMG and mixed followed by granulating the step-2 materials by using step-1 solution. The wet mass was dried to obtain granule, mixed well with extragranular MCC and talc and then filled into capsule or compressed into tablet.

### Example 9

| **Ingredients** | **Mg/Tablet** | **%W/W** |
|---|---|---|
| **Intragranular material** | | |
| Fingolimod HCl | 0.56 | 0.86 |
| Glycine | 22.72 | 34.95 |
| Micro crystalline cellulose (MCC) 101 | 30.22 | 46.49 |
| Polyvinyl pyrrolidone (PVP) k29/32 | 1.5 | 2.31 |
| Purified water | **QS** | |

| **Extra granular material** | | |
|---|---|---|
| Micro crystalline cellulose (MCC) 101 | 9 | 13.85 |
| Talc | 1 | 1.54 |
| **Total** | **65** | **100** |

**Procedure:** Fingolimod HCl and small amount of Glycine was dissolved in water. Remaining amount of Glycine, PVP K, MCC, was loaded in RMG and mixed followed by granulating the step-2 materials by using step-1 solution. The wet mass was dried to obtain granule, mixed well with extragranular MCC and talc and then filled into capsule or compressed into tablet.

## Claims

1. A stable pharmaceutical composition comprising fingolimod, a pharmaceutically acceptable salt thereof or a phosphate derivative thereof, and a zwitterion as a stabilizer, wherein the composition is free of sugar alcohol and wherein the zwitterion is an amino acid.

2. The composition according to claim 1 is in the form of capsules, powders, granules, tablets, pills, lozenges, sachets, suppositories.

3. The pharmaceutical composition according to claim 1 or 2, wherein the amino acid is selected from glycine, arginine, histidine, alanine, isoleucine, leucine, asparagine, lysine, aspartic acid, methionine, cysteine, phenylalanine, glutamic acid, threonine, glutamine, tryptophan, valine, ornithine, proline, selenocysteine, serine, tyrosine or a combination thereof.

4. The pharmaceutical composition according to claim 3, wherein the amino acid is selected from glycine, leucine or mixtures thereof.

5. The pharmaceutical composition according to claim 1, wherein the composition further comprises one or more pharmaceutically acceptable excipients selected from diluent, binder, disintegrant, surafactant, glidant, lubricant.

6. The pharmaceutical composition according to claim 5, wherein the diluent is selected from microcrystalline cellulose, powdered cellulose, lactose (anhydrous or monohydrate), compressible sugar, fructose, dextranes, or a mixture thereof, siliconised microcrystalline cellulose, calcium hydrogen phosphate, calcium carbonate, calcium lactate or mixtures thereof.

7. The pharmaceutical composition according to claim 5, wherein the binder is selected from polyvinyl pyrollidone, polyvinylpyrrolidone/vinyl acetate copolymer, polyvinyl alcohol, polymers of acrylic acid and its salts, starch, methyl cellulose, ethyl cellulose, hydroxyethyl cellulose, hydroxyl propyl cellulose, ethylhydroxyethylcellulose, hydroxypropyl methylcellulose, carboxymethyl cellulose etc., maltrin, sucrose solution, dextrose solution, acacia, tragacanth, locust bean gum, gelatine, guar gum, starch, pregelatinised starch, partially hydrolysed starch, alginates, xanthan or polymethacrylate, or mixtures thereof.

8. The pharmaceutical composition according to claim 5, wherein the disintegrant is selected from crospovidone, sodium starch glycolate, croscarmellose sodium, carboxymethylcellulose calcium or mixture thereof.

9. The pharmaceutical composition according to claim 4, wherein the mean particle size of glycine is less than 250µm and d₉₀ is less than 400µm.

10. The pharmaceutical composition according to claim 4, wherein the mean particle size of leucine is less than 350 µm and d₉₀ is less than 600µm.

11. A stable pharmaceutical composition according to claim 1 comprising at least one pharmaceutically acceptable excipient selected from
a) diluent selected from microcrystalline cellulose, lactose monohydrate or mixture thereof;
b) binder selected from hydroxypropyl cellulose or povidone;
c) lubricants selected from stearic acid, magnesium stearate or calcium stearate.
d) disintegrant selected from crospovidone, sodium starch glycolate, croscarmellose sodium,
e) glidant selected from colloidal silica or talc.

## Patentansprüche

1. Eine stabile pharmazeutische Zusammensetzung umfassend Fingolimod, ein pharmazeutisch akzeptables Salz davon oder ein Phosphat-Derivat davon sowie ein Zwitterion als Stabilisator, wobei die Zusammensetzung frei von Zuckeralkoholen ist und wobei das Zwitterion eine Aminosäure ist.

2. Pharmazeutische Zusammensetzung gemäß Anspruch 1, die in From von Kapseln, Pulvern, Granalien, Tabletten, Pillen, Lutschtabletten, Sachets oder Zäpfchen vorliegt.

3. Pharmazeutische Zusammensetzung gemäß Anspruch 1 oder 2, wobei die Aminosäure ausgewählt ist aus Glycin, Arginin, Histidin, Alanin, Isoleucin, Leucin, Asparagin, Lysin, Asparaginsäure, Methionin, Cystein, Phenylalanin, Glutaminsäure, Threonin, Glutamin, Tryptophan, Valin, Ornithin, Prolin, Selencystein, Serin, Tyrosin oder einer Kombination davon.

4. Pharmazeutische Zusammensetzung gemäß Anspruch 3, wobei die Aminosäure ausgewählt ist aus Glycin, Leucin oder einer Kombination davon.

5. Pharmazeutische Zusammensetzung gemäß Anspruch 1, wobei die pharmazeutische Zusammensetzung ferner einen oder mehrere pharmazeutisch akzeptable Hilfsstoffe ausgewählt aus Streckmitteln, Bindemitteln, Sprengmitteln, grenzflächenaktive Substanzen, Gleitmitteln und Schmiermitteln umfasst.

6. Pharmazeutische Zusammensetzung gemäß Anspruch 5, wobei das Streckmittel ausgewählt ist aus mikrokristalliner Cellulose, gepulverter Cellulose, Laktose (wasserfrei oder das Monohydrat), komprimierbarem Zucker, Fruktose, Dextranen oder Mischungen davon, silikonisierter mikrokristalliner Cellulose, Calciumhydrogenphosphat, Calciumcarbonat, Calciumlaktat oder Mischungen davon.

7. Pharmazeutische Zusammensetzung gemäß Anspruch 5, wobei das Bindemittel ausgewählt ist aus Polyvinylpyrrolidon, Polyvinylpyrrolidon/Vinylacetat-Copolymer, Polyvinylalkohol, Polymere der Acrylsäure und ihrer Salze, Stärke, Methylcellulose, Ethylcellulose, Hydroxyethylcellulose, Hydroxypropylcellulose, Ethylhydroxyethylcellulose, Hydroxypropylmethylcellulose, Carboxymethylcellulose, etc., Maltrin, Saccharose-Lösung, Dextrose-Lösung, Acacia, Tragacanth, Johannisbrotkernmehl, Gelatine, Guarkernmehl, Stärke, vorgekleisterter Stärke, teilweise hydrolysierter Stärke, Alginaten, Xanthan oder Polymethacrylaten oder Mischungen davon.

8. Pharmazeutische Zusammensetzung gemäß Anspruch 5, wobei das Sprengmittel ausgewählt ist aus Crospovidon, Natrium-Stärkeglycolat, Natrium-Croscarmellose, Calcium-Carboxymethylcellulose oder Mischungen davon.

9. Pharmazeutische Zusammensetzung gemäß Anspruch 4, wobei die mittlere Partikelgröße des Glycins unterhalb von 250 µm und der d90-Wert unterhalb von 400 µm liegt.

10. Pharmazeutische Zusammensetzung gemäß Anspruch 4, wobei die mittlere Partikelgröße des Leucins unterhalb von 350 µm und der d90-Wert unterhalb von 600 µm liegt.

11. Stabile pharmazeutische Zusammensetzung gemäß Anspruch 1 umfassend wenigstens einen pharmazeutisch akzeptablen Hilfsstoff ausgewählt aus
a. Streckmittel ausgewählt aus mikrokristalliner Cellulose, Laktose-Monohydrat oder Mischungen davon,
b. Bindemittel ausgewählt aus Hydroxypropylcellulose oder Povidon,
c. Schmiermittel ausgewählt aus Stearinsäure, Magnesiumstearat oder Calciumstearat,
d. Sprengmittel ausgewählt aus Crospovidon, Natrium-Stärkeglycolat, Natrium- Croscarmellose,
e. Gleitmitteln ausgewählt aus kolloidalem Siliziumdixid oder Talkum.

## Revendications

1. Composition pharmaceutique stable comprenant du fingolimod, un de ses sels pharmaceutiquement acceptables ou un de ses dérivés phosphatés, et un zwitterion utilisé en tant que stabilisant, **caractérisée en ce qu'**elle est dépourvue de sucre alcool et **en ce que** le zwitterion est un acide aminé.

2. Composition selon la revendication 1, **caractérisée en ce qu'**elle se présente sous la forme de gélules, poudres, granules, comprimés, pilules, pastilles, sachets, suppositoires.

3. Composition pharmaceutique selon la revendication 1 ou 2, **caractérisée en ce que** l'acide aminé est sélectionné parmi glycine, arginine, histidine, alanine, isoleucine, leucine, asparagine, lysine, acide aspartique, méthionine, cystéine, phénylalanine, acide glutamique, thréonine, glutamine, tryptophane, valine, ornithine, proline, sélénocystéine, sérine, tyrosine, ou un mélange de cex-ci.

4. Composition pharmaceutique selon la revendication 3, **caractérisée en ce que** l'acide aminé est sélectionné parmi glycine, leucine ou des mélanges de ceux-ci.

5. Composition pharmaceutique selon la revendication 1, **caractérisée en ce qu'**elle comprend en outre un ou plusieurs excipients pharmaceutiquement acceptables sélectionnés parmi diluant, liant, désintégrant, surfactant, agent de glissement, lubrifiant.

6. Composition pharmaceutique selon la revendication 5, **caractérisée en ce que** le diluant est sélectionné parmi cellulose microcristalline, poudre de cellulose, lactose (anhydre ou mono-hydraté), sucre compressible, fructose, dextrans, ou un mélange de ceux-ci, cellulose microcristalline siliconée, phosphate bicalcique, carbonate de calcium, lactate de calcium, ou des mélanges de ceux-ci.

7. Composition pharmaceutique selon la revendication 5, **caractérisée en ce que** le liant est sélectionné parmi polyvinylpyrrolidone, copolymère polyvinylpyrrolidone/acétate de vinyle, alcool polyvinylique, polymères de l'acide acrylique et leurs sels, amidon, méthylcellulose, éthylcellulose, hydroxyéthylcellulose, hydroxypropylcellulose, éthylhydroxyéthylcellulose, hydroxypropylméthylcellulose, carboxyméthylcellulose, etc., maltrin, une solution de saccharose, une solution de dextrose, acacia, gomme adragante, gomme de caroube, gélatine, gomme guar, amidon, amidon prégélatiné, amidon partiellement hydrolysé, alginates, xanthane ou polyméthacrylate, ou des mélanges de ceux-ci.

8. Composition pharmaceutique selon la revendication 5, **caractérisée en ce que** le désintégrant est sélectionné parmi crospovidone, glycolate sodique d'amidon, croscarmellose sodique, calcium de carboxyméthylcellulose, ou un mélange de ceux-ci.

9. Composition pharmaceutique selon la revendication 4, **caractérisée en ce que** le diamètre moyen des particules de glycine est inférieur à 250 µm et que le d90 est inférieur à 400 µm.

10. Composition pharmaceutique selon la revendication 4, **caractérisée en ce que** le diamètre moyen des particules de leucine est inférieur à 350 µm et que le d90 est inférieur à 600 µm.

11. Composition pharmaceutique stable selon la revendication 1 comprenant au moins un excipient pharmaceutiquement acceptable sélectionné parmi :
a) un diluant sélectionné parmi cellulose microcristalline, lactose mono-hydraté ou leur mélange ;
b) un liant sélectionné parmi hydroxypropylcellulose ou polyvidone ;
c) des lubrifiants sélectionnés parmi acide stéarique, stéarate de magnésium ou stéarate de calcium.
d) un désintégrant sélectionné parmi crospovidone, glycolate sodique d'amidon, croscarmellose sodique,
e) un agent de glissement sélectionné parmi silice colloïdale ou talc.
